# EUROPÄISCHE PATENTSCHRIFT

(11) **EP 3 606 454 B1**
(45) Veröffentlichungstag und Bekanntmachung des Hinweises auf die Patenterteilung: **16.08.2023**
(21) Anmeldenummer: 18716584.0
(22) Anmeldetag: 04.04.2018
(51) Int. Cl.: A61B 17/68, A61B 17/80, A61B 90/14, A61B 17/88

(54) **POSITIONIERVORRICHTUNG FÜR SCHÄDELKNOCHENABSCHNITTE, HERSTELLUNGSVERFAHREN FÜR POSITIONIERVORRICHTUNG UND SYSTEM AUS POSITIONIERVORRICHTUNG MIT BEFESTIGUNGSVORRICHTUNGEN**
POSITIONING DEVICE FOR CRANIAL BONE SECTIONS, PRODUCTION METHOD FOR POSITIONING DEVICE AND SYSTEM COMPRISING POSITIONING DEVICE HAVING FIXING DEVICES
POSITIONNEUR DE SECTIONS D'OS CRÂNIEN, PROCÉDÉ DE RÉALISATION D'UN POSITIONNEUR ET SYSTÈME DE POSITIONNEMENT À L'AIDE DE DISPOSITIFS DE FIXATION

(30) Priorität: 04.04.2017 DE 102017107259
(43) Veröffentlichungstag der Anmeldung: 12.02.2020
(73) Patentinhaber: Karl Leibinger Medizintechnik GmbH & Co. KG, 78570 Mühlheim (DE)
(72) Erfinder: REINAUER, Frank, 78570 Mühlheim (DE); GROM, Stefanie, 78570 Mühlheim (DE); GRIGORE, Anita, 78570 Mühlheim (DE); IRION, Veit, 78570 Mühlheim (DE); HABERL, Ernst-Johannes, 10719 Berlin (DE)
(74) Vertreter: Winter, Brandl - Partnerschaft mbB
(86) Internationale Anmeldenummer: PCT/EP2018/058636
(87) Internationale Veröffentlichungsnummer: WO 2018/185179

(56) Entgegenhaltungen:
- EP-A1- 2 522 289
- EP-A1- 2 792 333
- RU-C2- 2 638 894
- US-A1- 2012 110 828

## Beschreibung

Die Erfindung betrifft eine Positioniervorrichtung für Schädelknochenabschnitte zum Relativpositionieren der Schädelknochenabschnitte zueinander und zum Erhalten einer vordefinierten Gesamtform und Gesamtkontur, um eine Zielschädelform zu erreichen, mit einem zum Tragen der Schädelknochenabschnitte ausgelegten Hauptkörper. Weiterhin betrifft die Erfindung ein Herstellverfahren zum Erzeugen einer Positioniervorrichtung und eine Positioniervorrichtung an der zum Positionieren von Schädelknochenabschnitten ein oder mehrere Befestigungsvorrichtungen angebracht sind oder anbringbar sind, wobei die Befestigungsvorrichtung(en), ausgelegt ist/sind, um die Schädelknochenabschnitte temporär an der Positioniervorrichtung zu befestigen.

Zum Korrigieren von Schädeldeformierungen werden zumeist die deformierten Schädelknochenabschnitte durch einen chirurgischen Eingriff in eine undeformierte Form gebracht.

Aus dem Stand der Technik sind bereits Vorrichtungen zum Ausrichten (und Korrigieren) von deformierten Schädelknochenabschnitten bekannt. Unter anderem offenbart die EP 2 522 289 A1 ein dreidimensionales Modell eines menschlichen Schädels in Lebensgröße, umfassend einen Stirnteil, einen Scheitelteil und einen Hinterhauptteil, wobei es sterilisierbar ist. Eine solche Positioniervorrichtung wird vor allem eingesetzt, um Störungen des Schädelwachstums korrigieren zu können. Dabei werden die deformierten Schädelknochenabschnitte durch einen operativen Eingriff repositioniert und an eine Sollschädelform angepasst.

Der Stand der Technik hat jedoch den Nachteil, dass zu der Reposition der Schädelknochenabschnitte Positioniervorrichtungen verwendet werden, die das Modell eines durchschnittlichen Kinderschädels darstellen. Aber auch bei einer großen Auswahl von Schädelmodellen stimmt der individuelle Sollschädel nur teilweise oder größtenteils mit dem Durchschnittsmodell überein. Da die Abweichungen eines Individualschädels jedoch von einem nächstähnlichen, durchschnittlichen Modell sehr groß sind, kann kein optimales Ergebnis bei der Modellierung der Schädelknochenabschnitte erzielt werden. Der Erfolg der Operation ist dann von dem Geschick und dem Können des Operateurs abhängig.

US 2012/0110828 A1 offenbart eine Positioniervorrichtung gemäß der Präambel des Anspruchs 1 und ein Herstellverfahren gemäß der Präambel des Anspruchs 9.

Es ist also die Aufgabe der Erfindung, die Nachteile aus dem Stand der Technik zu vermeiden oder wenigstens zu mildern. Insbesondere soll eine Positioniervorrichtung entwickelt werden, die für den jeweiligen individuellen Schädel optimal angepasst ist, so dass die Modellierung der Schädelknochen erheblich erleichtert wird, da der Operateur die Schädelknochen nicht mehr manuell anpassen muss.

Die Aufgabe der Erfindung wird erfindungsgemäß dadurch gelöst, dass der Hauptkörper der Positioniervorrichtung, der basierend auf individuellen Patientendaten, die spezifisch für den zu behandelnden Patienten sind, und ergänzt durch modifizierte, anatomische Daten, zum Beispiel in einem generischen Verfahrensschritt, hergestellt ist und eine Tragstruktur aufweist, eine sich kreuzende Linienstruktur besitzt.

Dabei wird die Zielschädelform definiert, dadurch dass auf Basis von geometrischen Daten von gesunden, nicht verformten Schädeln eine durchschnittliche Form sowie die meisten/alle charakteristischen Variationen der Form berechnet werden. Zugehörige geometrische Punkte jeder Schädeloberfläche werden bestimmt, indem anatomisch zugehörige Regionen von einer Schädelform auf eine andere übertragen/projiziert/angewendet werden, wobei metrische Verformungen von deformierten Stellen minimiert werden. Durch diese modifizierten, anatomischen Daten, die durch Anpassung der statistisch erfassten, unverformten Schädelformen auf verformte Stellen des Schädels mit Berücksichtigung der Variationen der Schädelform erzielt werden, kann also eine individuelle Soll-Schädelform errechnet werden, die die verformten Stellen des Schädels so anpasst und korrigiert, dass eine unverformte Schädelform entsteht. Unter einem "Modifizieren" wird also (auch) ein "Weiterverarbeiten" verstanden. Es werden also Referenzpunkte an einem vergleichbaren unverformten Schädel ausgewählt, als Korrekturwerte ermittelt und dann in den spezifischen Schädel weiterverarbeitend eingearbeitet.

Dies hat den Vorteil, dass die repositionierten Schädelknochenabschnitte mit Hilfe der Positioniervorrichtung einfach in eine individuelle Soll-Schädelform gebracht werden können. Die entnommenen Schädelknochenabschnitte können also einfach an die Außenseite oder Innenseite der Positioniervorrichtung angelegt und an dessen Form angepasst werden, um ein optimales Ergebnis zu erzielen. Es ist nicht mehr nötig, manuell individuelle Anpassungen und Korrekturen der Abweichungen von einem auf einem durchschnittlichen Schädel basierenden Modell vorzunehmen, da die individuelle Soll-Schädelform/Zielschädelform bereits zur Erstellung des Hauptkörpers der Positioniervorrichtung geeignet berücksichtigt wird. Dabei ist es von Vorteil, wenn die personengebundenen Patientendaten, die den Schädel betreffen, es zulassen, dass die individuelle Soll-Schädelform berechnet wird.

Vorteilhafte Ausführungsformen sind in den Unteransprüchen beansprucht und werden nachfolgend näher erläutert.

Auch ist es dabei vorteilhaft, wenn der Rand geschlossen ausgestaltet ist, da so die Stabilität der Positioniervorrichtung erhöht wird. Der Rand dient also als eine Grundplatte/Grundumriss und als Begrenzung für die Tragstruktur, an die die Schädelknochenabschnitte zur Modellierung angelegt werden.

Ferner zeichnet sich ein günstiges Ausführungsbeispiel dadurch aus, dass die Tragstruktur auf nur einer Seite einer gedachten, durch den Rand verlaufenden Fläche oder Ebene angeordnet ist. Dadurch wird genau festgelegt, in welchem Bereich die Schädelknochenabschnitte angeordnet werden müssen.

Weiterhin ist es zweckmäßig, wenn die Fläche durch eine gemeinsame Verschneidung mehrerer Ebenen vorgegeben ist. So hat der Rand eine nahezu ebene Außenkante, die auch als Orientierung für eine horizontale Ausrichtung der Positioniervorrichtung verwendet werden kann.

Zudem ist es vorteilhaft, wenn von dem Hauptkörper, etwa im Bereich des Randes und/oder der Tragstruktur, ein Griffteil/Halteelement nach außen absteht, um die Positioniervorrichtung zu halten, befestigen und/oder einzuspannen. Dadurch wird das Halten, insbesondere während der Operation, erleichtert. Es wird auch ermöglicht, dass die Positioniervorrichtung fest fixiert werden kann, so dass sie nicht verkippen kann.

Wenn das Griffteil/Halteelement näherungsweise oder exakt rechtwinklig vom Hauptkörper an der gemeinsamen Kontaktstelle gemessen absteht, dann ist es besonders günstig, das Griffteil/Halteelement einzuspannen. Insbesondere kann das Griffteil/Halteelement in eine Horizontalrichtung abstehen, da dies zusätzlich die Orientierung zum Anordnen der Schädelknochenabschnitte verbessert.

Auch ist es von Vorteil, wenn der Rand über den Umfang gesehen eine etwa gleichbleibende Dicke und/oder eine etwa gleichbleibende Höhe besitzt, damit die Stabilität über den Umfang gesehen konstant ist und die Spannungen in dem Rand gleichmäßig sind. Die Dicke und/oder die Höhe können jedoch auch an einigen über den Umfang verteilten Stellen des Rands, beispielsweise im Bereich des Halteelements, größer oder kleiner sein.

Ein günstiges Ausführungsbeispiel zeichnet sich auch dadurch aus, dass der Randbereich im Bereich des abstehenden Griffteils/Halteelements eine Erhöhung und/oder eine Verdickung besitzt. Dadurch kann das Griffteil/Halteelement stabiler an den Hauptkörper der Positioniervorrichtung angebunden werden und somit eine bessere Kraftübertragung gewährleisten.

Zudem ist es zweckmäßig, wenn die Tragstruktur netzartig oder gitterartig ausgeformt ist, was vorteilhafterweise zum einen das Gewicht der Tragstruktur senkt, was die Handhabung verbessert, und zum anderen ein Durchgreifen eines Befestigungselements zum temporären Fixieren der Schädelknochenabschnitte an der Positioniervorrichtung ermöglicht.

Ferner ist es vorteilhaft, wenn die Tragstruktur eine Gitterstruktur oder Netzstruktur besitzt, was bei geringem Gewicht eine optimierte Festigkeit der Tragstruktur sicherstellt.

Auch kann der Hauptkörper als konvexe Hülle ausgebildet sein und/oder eine vorzugsweise gleichbleibende Dicke besitzen. Insbesondere ist es wichtig, dass die Seite oder Fläche, insbesondere die Innenseite/Innenfläche, des Hauptkörpers, an der die Schädelknochenabschnitte angelegt werden, konvex ausgestaltet ist, da sie sich so einer typischen Schädelform anpasst. Insbesondere ist es von Vorteil, wenn der Hauptkörper etwa halbkugelförmig ausgebildet ist.

Der Hauptkörper der Positioniervorrichtung kann helmartig ausgebildet sein, d. h., dass sich die Innenseite des Hauptkörpers an die Außenseite des Schädels anschmiegt. So können die Schädelknochenabschnitte also auf der Innenseite angeordnet werden und in die Soll-Schädelform gebracht werden.

Ferner ist es zweckmäßig, wenn die Tragstruktur eine Vielzahl von Aussparungen oder Durchgangslöchern besitzt, die durch Stege voneinander räumlich getrennt sind. Diese Aussparungen oder Durchgangslöcher ermöglichen also vorteilhafterweise ein Durchgreifen (für die Befestigungselemente) durch die Tragstruktur, was insbesondere zum Befestigen der Schädelknochenabschnitte an der Tragstruktur von Bedeutung ist.

Es ist von Vorteil, wenn die Stege eine Vieleckform, beispielsweise ein regelmäßiges Polygon definierend, wie etwa eine Wabenstruktur und/oder eine Sechseckform mit beispielsweise gleichlangen Kanten oder eine Lochgeometrie mit durchgängig gerundeter Außenkontur ausbilden. So ermöglichen die Stege, dass ein Teil des Befestigungselements zum Fixieren der Schädelknochenabschnitte an der Tragstruktur aufliegen kann und ein anderer Teil des Befestigungselements durch die Aussparungen hindurchgreifen kann.

Weiterhin ist es zweckmäßig, wenn sich eine Außenkontur aller oder einiger Durchgangslöcher in ihrer Geometrie und/oder Fläche entspricht. So können also die gleichen Befestigungselemente an verschiedenen/an allen Stellen auf der Tragstruktur eingesetzt werden.

Ferner zeichnet sich ein günstiges Ausführungsbeispiel dadurch aus, dass die Durchgangslöcher eine gleichmäßige Konfiguration/Ausprägung haben. Dadurch ist es also nicht von Bedeutung, in welcher Ausrichtung, insbesondere in welcher Position bei einer Drehung um eine in Radialrichtung der Positioniervorrichtung abstehende Richtung, die Befestigungselemente durch die Durchgangslöcher gesteckt werden.

Auch ist es möglich, wenn die Durchgangslöcher eine kreisförmige, rundliche, elliptische oder ovale Fläche besitzen, dass sie dann vorteilhafte Ausführungsformen eines darauf angepassten Befestigungselements sicher aufnehmen.

Zusätzlich ist es zweckmäßig, wenn die Stege eine gleichbleibende und/oder gleichartige Dicke besitzen. So wird sichergestellt, dass es nicht zu lokalen Schwachstellen oder Spannungsspitzen in den Stegen kommt.

Ferner ist es von Vorteil, wenn die Stege einen trapezförmigen oder dreieckigen Querschnitt besitzen, was die konvexe Ausbildung der Tragstruktur zusätzlich unterstützt.

Auch können die Stege einen viereckigen Querschnitt besitzen, wobei zwei überwiegend in Radialrichtung Hauptbegrenzungsflächen aufeinander zulaufen und zwei diese beiden Hauptbegrenzungsflächen begrenzenden Nebenbegrenzungsflächen parallel zueinander angeordnet sind. So bilden die Stege also auf der Innenseite der Tragstruktur eine größere Fläche als auf der Außenfläche aus, was vorteilhafterweise eine größere Auflagefläche für die Schädelknochenabschnitte bietet.

Auch ist es vorteilhaft, wenn die beiden Hauptbegrenzungsflächen von innen nach außen gesehen aufeinander zulaufen, etwa unter Ausbildung eines Winkels zwischen 20 ° und 45 °, beispielswiese 30 °, 33 ° und 35 °.

Zudem ist es zweckmäßig, wenn mindestens einer der Stege oder alle Stege ein Längen-/Breitenverhältnis von 10/3 ± 10 Prozent besitzen und/oder ein Längen-/Dickenverhältnis von 10/3 ± 10 Prozent besitzen. So wird eine günstige Kraftübertragung zwischen den Stegen sichergestellt.

Grundsätzlich zeichnet sich ein günstiges Ausführungsbeispiel dadurch aus, dass der Rand eine Höhe besitzt, die zwischen 2- bis 4- mal so groß ist, wie die Dicke oder Breite der Stege. Durch diese zusätzliche Versteifung am Rand der Tragstruktur wird also eine Beibehaltung der Form sichergestellt.

Auch ist es vorteilhaft, wenn die Positioniervorrichtung an der Hinterseite eine Schräge aufweist. Dabei ist die Hinterseite die Seite, an welcher die Schädelknochenabschnitte, die normalerweise an dem Hinterkopf liegen, angeordnet werden. Diese Schräge erleichtert die Handhabung und die Anordnung der Schädelknochenabschnitte auf der Innenseite der Positioniervorrichtung.

Insbesondere ist es vorteilhaft, wenn die Schräge in einem Winkel von 30 bis 60 °, bevorzugt 40 bis 50 °, weiter bevorzugt etwa 25 °, absteht.

Ferner ist es zweckmäßig, wenn die Positioniervorrichtung eine Mittelinienmarkierung aufweist. Dadurch kann der Operateur gut erkennen, wie die Schädelknochenabschnitte anzuordnen sind, da er eine bessere Orientierung bekommt.

Weiter ist es von Vorteil, wenn die Mittellinienmarkierung durch einen von einem ersten Bereich des Randes zu einem gegenüberliegenden Bereich des Randes verlaufenden Steg oder eine entsprechend verlaufende Aussparung ausgebildet ist. Vorzugsweise verläuft die Mittellinienmarkierung von der Vorderseite zur Hinterseite, so dass die Scheitellinie der Schädelknochenabschnitt markiert wird.

Auch ist es zweckmäßig, wenn der Hauptkörper vollständig oder teilweise aus Kunststoff besteht. Kunststoff bietet sich insbesondere bei medizinischen Anwendungen an, da viele Kunststoffe sterilisierbar sind und in vielen Bereichen eingesetzt werden können.

Eine weitere Bevorzugung ist, wenn der Hauptkörper vollständig oder teilweise aus Polyamid besteht. Polyamid weist besonders günstige Eigenschaften hinsichtlich Kosten und Gewicht auf und bietet sich für diese Anwendung an.

Auch ist es vorteilhaft, wenn die Positioniervorrichtung eine Anti-Rutsch-Beschichtung aufweist, da sie so besser gegriffen oder gehandhabt werden kann und ein unbeabsichtigtes Entgleiten wirksam verhindert wird.

Die Aufgabe der Erfindung wird auch durch ein erfindungsgemäßes Herstellverfahren zum Erzeugen einer Positioniervorrichtung, die vorbereitet ist, um an ihr Schädelknochenabschnitte temporär zu befestigen, wobei in einem Herstellschritt, basierend auf Geometriedaten, die repräsentativ für die Schädelknochengeometrie des bestimmten, zu behandelnden Patienten sind, ein Hauptkörper der Positioniervorrichtung geschaffen/erzeugt/erstellt wird, der eine Tragstruktur aufweist, gelöst, indem die Tragstruktur eine sich kreuzende Linienstruktur besitzt.

Dabei ist es von Vorteil, wenn in einem dem Herstellverfahren vorgeschalteten Referenzschritt/Messschritt die Geometriedaten des Patienten erfasst und optional weiterverarbeitet werden.

Auch können Geometriedaten im Referenzschritt mit photometrischen Mitteln und/oder durch Photometrie erfasst werden, was vorteilhafterweise eine Strahlenbelastung für den Patienten vermeidet und somit insbesondere bei der Anwendung für Kinder empfehlenswert ist.

Insbesondere werden kephalometrische Punkte als Messpunkte für die Geometriedaten verwendet, da sich aus wenigen kephalometrischen Punkten durch eine Berechnungsformel eine gesamte, typische Sollschädelform errechnen lässt. Dadurch ist man nicht mehr auf Durchschnittswerte oder statistische Auswertungen angewiesen, sondern kann eine individuelle, perfekt angepasste Sollschädelform errechnen.

Es ist zweckmäßig, wenn von den kephalometrischen Punkten wenigstens einer von Labella, Opisthocranium, Eurion, Orbitale, Nasion, Pogonion, Gnation, Menton, Gonion, Bregma, Lambda, Zygion, Porion, Mastoidale, Basion, Inion oder Vertex gemessen wird. Diese Punkte dienen als Ausgangspunkte für die Berechnungsformel zur Berechnung einer Sollschädelform.

Es ist von Vorteil, wenn auf Basis der Geometriedaten ein virtuelles Sollschädelmodell (des Schädels mit einer gewünschten Form) berechnet/erstellt wird. Das Sollschädelmodell entspricht also einem Modell eines Schädels, der nicht deformiert ist.

Außerdem ist es vorteilhaft, wenn das virtuelle Sollschädelmodell genutzt wird, um die Innenfläche der Positioniervorrichtung so auszuformen, dass diese mit der Außenfläche des Sollschädels exakt übereinstimmt. Dadurch wir also vorteilhafterweise ermöglicht, dass ein deformierter Schädelabschnitt durch Anlegen an der Positioniervorrichtung in die gewünschte Form gebracht wird. Infolgedessen ist es vorteilhaft, wenn die Positioniervorrichtung so ausgebildet wird, dass die Innenfläche der Positioniervorrichtung einer Außenfläche eines SollSchädels/Zielschädels entspricht.

Auch ist es zielführend, wenn für das virtuelle Soll-Schädelmodell repräsentative Geometriewerte durch einen Überführungsschritt in Fertigungsgeometriedaten umgewandelt werden, basierend auf denen in einem Fertigungsschritt, die Positioniervorrichtung, also der Hauptkörper und beispielsweise auch das Griffteil/Halteelement, hergestellt wird.

Es ist also von Vorteil, wenn zuerst die Geometriedaten erfasst werden, dann darauf basierend die Geometriewerte errechnet werden, also die Soll-Schädelform, und danach die Fertigungsgeometriedaten, die einem "Helm" bzw. einer Innenseite des Hauptkörpers oder einer Außenseite des Sollschädelmodells entsprechen, berechnet werden. Aus diesen Fertigungsgeometriedaten wird dann die generative Positioniervorrichtung gestellt.

Weiterhin ist es von Vorteil, wenn die Positioniervorrichtung durch additive Fertigung, beispielsweise durch 3D-Druck, hergestellt wird. So kann innerhalb kürzester Zeit basierend auf den Fertigungsgeometriedaten jegliche Form zu annehmbaren Kosten erstellt/gedruckt werden.

Die Aufgabe der Erfindung wird auch durch eine solche Positioniervorrichtung zusammen mit einer Schädelknochenbefestigungsvorrichtung/Befestigungsvorrichtung, die ausgelegt ist, um die Schädelknochenabschnitte temporär an der Positioniervorrichtung zu befestigen, gelöst. Dabei ist es vorteilhaft, wenn die Schädelknochenbefestigungsvorrichtung/Befestigungsvorrichtung zum temporären Befestigen eines Schädelknochenabschnitts an dem Hauptkörper der Positioniervorrichtung ausgelegt ist.

Die Befestigungsvorrichtung besitzt vorzugsweise einen Fixierelementabschnitt, der zum einseitigen, direkten Kontaktieren des Schädelknochenabschnittes oder mehrerer Schädelknochenabschnitte an der einen Seite ausgelegt ist, um bei Zusammenwirken mit einem Gegenabschnitt auf der anderen Seite des Schädelknochenabschnitts oder der Schädelknochenabschnitte diesen oder diese am Hauptkörper festzulegen. Dabei sind der Fixierelementabschnitt und der Gegenabschnitt ausgelegt, um im Befestigungszustand ineinander zu greifen.

Die Befestigungsvorrichtung kann zumindest zweiteilig aufgebaut sein, wobei ein Teil der Befestigungsvorrichtung den Fixierelementabschnitt, der einen Stift besitzt, bildet, wobei der Fixierelementabschnitt bemessen ist, um von der einen Seite des Schädelknochenabschnitts auf die andere Seite des Schädelknochenabschnitts sich im Befestigungszustand zu erstrecken, und wobei ein anderer Teil der Befestigungsvorrichtung den Gegenabschnitt bildet, der ausgelegt ist, um den Fixierelementabschnitt so aufzunehmen, dass der Schädelknochenabschnitt an der Positioniervorrichtung befestigt wird.

Der Gegenabschnitt besitzt einen Griffbereich und einen Abstützbereich. Der Fixierelementabschnitt besitzt einen Griffbereich, einen Stützbereich und einen Koppelbereich mit einem Gewinde und einem Stift.

Die erfindungsgemäße Positioniervorrichtung kann in einem Verfahren zur Behandlung eines Patienten, wie eines menschlichen Kindes, im Bereich des Kopfes verwendet werden.

Dabei ist es vorteilhaft, wenn zuerst die Geometriedaten des Patienten erfasst werden, dann die Geometriewerte für das virtuelle Soll-Schädelmodell berechnet und darauf basierend die Fertigungsgeometriedaten für die Positioniervorrichtung errechnet werden.

Es ist vorteilhaft, die Positioniervorrichtung in einem generativen Verfahren vor der Operation zu erzeugen. So kann die Operation innerhalb möglichst kurzer Zeit durchgeführt werden.

Zusätzlich ist es zweckmäßig, wenn in einem Operationsschritt die deformierten Schädelknochenabschnitte entnommen werden. Auch ist es von Vorteil, die entnommenen Schädelknochenabschnitte in der Positioniervorrichtung anzuordnen und zueinander auszurichten, so dass sie etwa eine geschlossene Fläche bilden.

Es ist zweckmäßig, die ausgerichteten Schädelknochenabschnitte mit einer oder mehreren Schädelknochenbefestigungsvorrichtungen/Befestigungsvorrichtungen an dem Hauptkörper der Positioniervorrichtung zu befestigen.

Danach können die in die angepasste Form gebrachten Schädelknochenabschnitte über ein etwa streifenförmiges Implantat aneinander fixiert werden, so dass sie die Position halten. Danach können die Schädelknochenbefestigungsvorrichtungen wieder von der Positioniervorrichtung und den Schädelknochenabschnitten gelöst werden.

Anschließend können die aneinander befestigten Schädelknochenabschnitte aus der Positioniervorrichtung herausgenommen werden und dann in den Schädel zurück implantiert werden.

Die Erfindung wird nachfolgend mit Hilfe einer Zeichnung erläutert. Es zeigen:
Fig. 1 eine perspektivische Ansicht einer erfindungsgemäßen Positioniervorrichtung mit einer Gitterstruktur in einem ersten Ausführungsbeispiel und einer Mittellinienmarkierung,
Fig. 2 eine Seitenansicht der Positioniervorrichtung in dem ersten Ausführungsbeispiel der Gitterstruktur,
Fig. 3 eine perspektivische Ansicht einer für ein erfindungsgemäßes System eingesetzte Schädelbefestigungsvorrichtung mit einem Gegenabschnitt und einem Fixierelementabschnitt,
Fig. 4 eine perspektivische Ansicht der Schädelknochenbefestigungsvorrichtung mit dem Fixierelementabschnitt, der in den Gegenabschnitt eingeschraubt ist,
Fig. 5 eine perspektivische Ansicht des Gegenabschnitts mit einem Griffbereich und einem Abstützbereich,
Fig. 6 eine perspektivische Ansicht des Fixierelementabschnitts mit einem Griffbereich, einem Stützbereich und einem Koppelbereich, und
Fig. 7 die Schädelbefestigungsvorrichtung in einer perspektivischen Ansicht.

Die Figuren sind lediglich schematischer Natur und dienen ausschließlich dem Verständnis der Erfindung. Die gleichen Elemente sind mit denselben Bezugszeichen versehen.

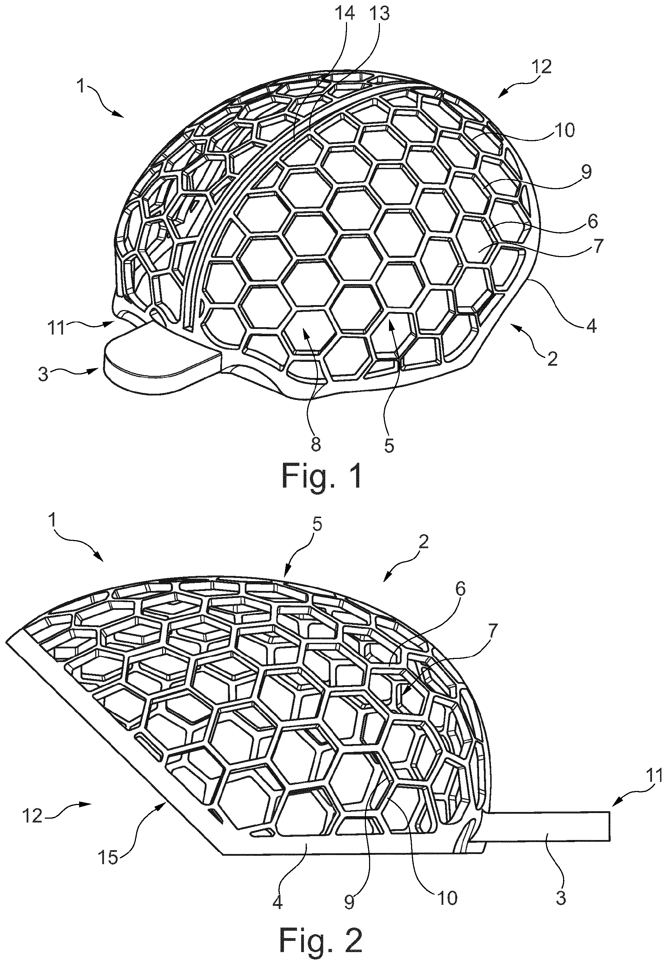

Fig. 1 zeigt eine Positioniervorrichtung 1 für Schädelknochenabschnitte. Die Positioniervorrichtung 1 ist ausgelegt, um die Schädelknochenabschnitte zueinander relativ zu positionieren und um eine vordefinierte Gesamtform und Gesamtkontur der Schädelknochenabschnitte zu erhalten oder zu schaffen. Die Positioniervorrichtung 1 besitzt einen Hauptkörper 2, an dem die Schädelknochenabschnitte angelegt werden. Außerdem besitzt die Positioniervorrichtung 1 ein Griffteil/Halteelement 3, das seitlich, etwa in einem rechten Winkel von einem Rand 4 der Positioniervorrichtung 1 absteht. Der Rand 4 ist Teil des Hauptkörpers 2 und begrenzt umlaufend eine Tragstruktur 5, die netzartig/gitterartig ausgebildet ist und etwa eine gedachte Halbkugelform umgibt.

Die Tragstruktur 5 ist eine konvexe Hülle und besteht aus Stegen 6, die mehrere Aussparungen/Durchgangslöcher 7 begrenzen. Die Aussparungen haben eine sechseckförmige Fläche und bilden eine Art Wabenform 8 aus. Die Aussparungen 7 sind gleichmäßig über die Tragstruktur 5 verteilt und werden nur von den Stegen 6 voneinander getrennt. Die Tragstruktur 5 bildet eine runde, etwa halbkugelförmige Form aus. Die Tragstruktur 5 erstreckt sich nur zu einer Seite des Randes 4, wobei der Rand eine umlaufende, geschlossene Kante bildet.

Die Stege 6 sind von konstanter Dicke und haben einen viereckigen, insbesondere einen trapezförmigen Querschnitt. Das heißt, dass die Stege 6 vier Flächen aufweisen, von denen zwei die Hauptbegrenzungsflächen 9 und zwei Nebenbegrenzungsflächen 10 sind. Die Hauptbegrenzungsflächen 9 erstrecken sich etwa in eine radiale Richtung der Positioniervorrichtung 1 und laufen von innen nach außen aufeinander zu. Die Nebenbegrenzungsflächen 10, die sich in Umfangsrichtung erstrecken, sind etwa parallel zueinander angeordnet.

Von einer Vorderseite 11 der Positioniervorrichtung 1 zu einer Hinterseite 12 der Positioniervorrichtung 1 erstreckt sich eine Mittellinienmarkierung 13. Die Mittellinienmarkierung 13 ist als durchgängige Aussparung 14, die sich von der einen Seite des Randes 4 zu einer gegenüberliegenden Seite des Randes 4 erstreckt. Die Vorderseite 11 ist die Seite der Positioniervorrichtung 1, die die Vorderseite des Kopfes ausbilden würde, wenn die Positioniervorrichtung 1 helmartig über einen Schädel gesetzt würde. Demnach entspricht die Form der Hinterseite 12 der Positioniervorrichtung 1 der Hinterkopfform.

Im Anschluss an die Aussparung 14 der Mittellinienmarkierung 13 geht die Tragstruktur 5 wieder in ihre gitterartig/netzartig/wabenartig ausgebildete Struktur mit den Stegen 6 und den Aussparungen 7 über. Die Tragstruktur 5 bildet an der Hinterseite 12 eine Schräge 15 aus, so dass die Tragstruktur 5 keine vollständige Halbkugel umschließt. Der Rand 4 bildet also in einer Seitenansicht (vergleiche Fig. 2) einen stumpfen Winkel aus, innerhalb welchem die Tragstruktur 5 angeordnet ist. Die Schräge 15 steht etwa in einem Winkel von 45 ° zu einer Horizontalen ab.

An der Vorderseite 11 der Positioniervorrichtung 1 schließt sich das Halteelement 3 an den Rand 4 an. Das Halteelement 3 steht in Horizontalrichtung nach außen von dem Hauptkörper 2 ab. Das Haltelement 3 ist mit einer konstanten Dicke ausgebildet. Das Halteelement 3 bildet eine Fläche aus, die ein Rechteck mit zwei an der Außenseite liegenden, abgerundeten Kante ist. An der Stelle des Randes 4, an dem das Halteelement 3 absteht, ist die Dicke/Höhe des Randes 4 im Vergleich zu dem restlichen Randbereich erhöht. Insgesamt ist der Hauptkörper 2 als eine Art konvexe Hülle ausgebildet, die eine Dicke von etwa 2 bis 5 mm aufweist.

Die Positioniervorrichtung 1 wird aufgrund von individuellen Patientendaten erzeugt, indem Vergleichsmesspunkte an dem Schädel des Patienten erfasst werden und darauf basierend eine virtuelle Sollschädelform errechnet wird, und diese wiederum für Berechnung von Fertigungsgeometriedaten, die der Positioniervorrichtung 1 entsprechend verwendet werden. Dabei ist die Positioniervorrichtung 1 als Hülle/Helm, die sich an die Sollschädelform außen anschmiegt, ausgebildet, so dass die Innenseite der Positioniervorrichtung 1 zur Anpassung der Form der Schädelknochenabschnitte verwendet werden kann.

Fig. 2 zeigt eine Seitenansicht der Positioniervorrichtung 1. Hier ist besonders gut zu erkennen, dass der Rand hier teilweise sich in eine horizontale Richtung erstreckt und dann in einem Winkel von etwa 45 ° nach oben abknickt.

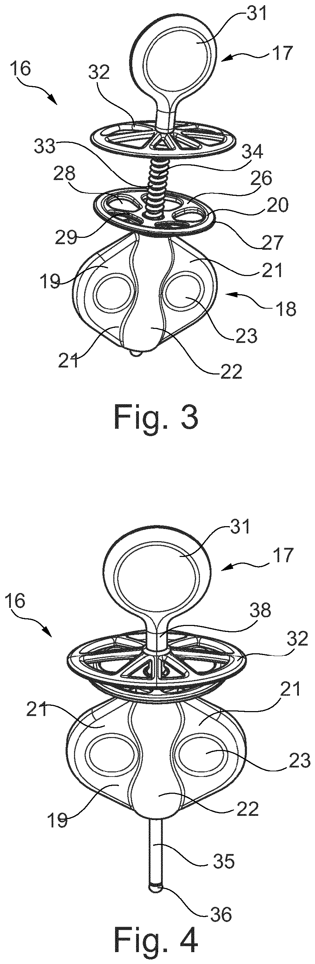

Fig. 3 zeigt eine Schädelknochenbefestigungsvorrichtung 16, die zum temporären Befestigen eines Schädelknochenabschnitts an dem Hauptkörper 2 der Positioniervorrichtung 1 ausgelegt ist. Die Schädelknochenbefestigungsvorrichtung 16 weist einen Fixierelementabschnitt 17 und einen Gegenabschnitt 18 auf. Der Fixierelementabschnitt 17 ist stiftartig ausgebildet und zum Kontaktieren des Schädelknochenabschnitts oder mehrerer Schädelknochenabschnitte auf der einen Seite ausgelegt, während er mit dem Gegenabschnitt 18, der auf der anderen Seite des Schädelknochenabschnitts oder der Schädelknochenabschnitte angeordnet ist, zusammenwirkt.

Der Gegenabschnitt 18 weist einen Griffbereich 19 und einen Abstützbereich 20 auf. Der Griffbereich 19 ist plattenartig ausgebildet und weist zwei Flügel 21 auf, die von beiden Seiten einer Verdickung 22 in derselben Ebene abstehen. Auf den Flügeln 21 ist jeweils eine runde, kreisförmige Vertiefung 23 ausgebildet. Die beiden Vertiefungen 23 sind zu beiden Seiten der Verdickung 22 in gleichem Abstand angeordnet. Die Verdickung 22 ist in einer Längsrichtung des Griffbereichs 19 angeordnet. Innerhalb der Verdickung 22 ist eine Aussparung 24 nach Art einer Durchgangsbohrung 25 in Längsrichtung ausgebildet.

Die Verdickung 23 ist von nicht konstanter Dicke, so dass sie in den beiden in Längsrichtung angeordneten Randbereichen einen größeren Durchmesser aufweist als in dem in Längsrichtung angeordneten Mittenbereich. Der Griffbereich 19 des Gegenabschnitts 18 weist eine Beschichtung auf, die besonders griffig, also antirutschend, fungiert. Der Griffbereich 19 des Gegenabschnitts 18 geht in den Abstützbereich 20 des Gegenabschnitts 18 über.

Der Abstützbereich 20 erstreckt sich etwa tellerartig in eine Ebene, die orthogonal zu der Längsrichtung des Gegenabschnitts 18 ist. Der Abstützbereich 20 ist also so ausgebildet, dass er eine konkave Fläche 26 und eine konvexe Fläche 27 aufweist, die zusammen eine konvexe Hülle des Abstützbereiches 20 ausbilden. Die konvexe Fläche 27 des Abstützbereichs 20 ist die Seite, die dem Griffbereich 19 zugewandt ist. Dagegen ist die konkave Fläche 26 die Fläche des Abstützbereichs 20, die von dem Griffbereich 19 abgewandt ist, also dem Fixierelementabschnitt 17 und in montiertem Zustand dem Schädelknochenabschnitt oder der Positioniervorrichtung 1 zugewandt ist.

Der Abstützbereich 20 ist rund, insbesondere oval, ausgebildet und weist Aussparungen 28 auf, die von Stegen 29 voneinander getrennt sind. In der Durchgangsbohrung 25 innerhalb der Verdickung 22 des Griffbereichs 19 und des Abstützbereichs 20 ist ein Innengewinde 30 eingebracht. Das Innengewinde 30 kann sich über die gesamte Länge der Durchgangsbohrung 25 oder nur über einen Bereich der Durchgangsbohrung 25 erstrecken.

In den Gegenabschnitt 18 der Schädelknochenbefestigungsvorrichtung 16 greift der Fixierelementabschnitt 17 im montierten Zustand ein. Der Fixierelementabschnitt 17 besteht aus einem Griffbereich 31, der über einen Steg 38 in einen Stützbereich 32 übergeht, wobei an den Stützbereich 32 ein Koppelbereich 33 anschließt. Der Koppelbereich 33 besteht aus einem Gewindebereich 34 und einem gewindelosen Endstift 35, der mit einer Verdickung 36 abschließt. Der Gewindebereich 34 weist ein Außengewinde auf, das so auf den Gegenabschnitt 18 abgestimmt ist, dass das Außengewinde des Gewindebereichs 34 in das Innengewinde 30 in der Durchgangsbohrung 25 eingreifen kann.

In Fig. 4 ist die Schädelknochenbefestigungsvorrichtung 16 in einem Zustand dargestellt, in dem der Fixierelementabschnitt 17 mit seinem Gewindebereich 34 in das Innengewinde 30 des Gegenabschnitts 18 eingeschraubt ist. Zwischen dem Abstützbereich 20 des Gegenabschnitts 18 und dem Stützbereich 32 des Fixierelementabschnitts 17 wird im Einsatzfall der Schädelknochenabschnitt an der Positioniervorrichtung 1 festgelegt.

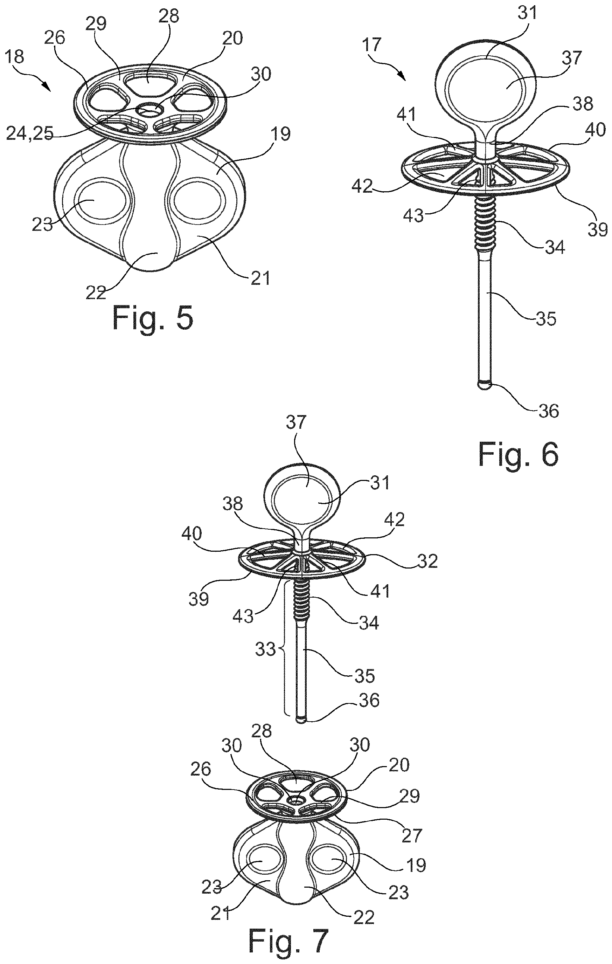

Fig. 5 zeigt eine vergrößerte Ansicht des Gegenabschnitts 18. Hier ist besonders gut zu erkennen, dass das Innengewinde 30 in der Durchgangsbohrung 25 ausgebildet ist. Der Gegenabschnitt 18 wird entweder auf der Innenseite der Positioniervorrichtung 1 angeordnet oder auf der Außenseite der Positioniervorrichtung 1. Da die Schädelknochenabschnitte auf der Innenseite der Positioniervorrichtung 1 angeordnet werden, kontaktiert der Gegenabschnitt 18 entweder auf der Innenseite die Schädelknochenabschnitte, insbesondere mit seinem Abstützbereich 20, oder auf der Außenseite die Gitterstruktur/Tragstruktur 5 der Positioniervorrichtung 1.

Fig. 6 zeigt eine vergrößerte Ansicht des Fixierelementabschnitts 17. Dabei geht der Griffbereich 31, der eine mittig angeordnete Vertiefung 37 aufweist, über den kleinen Steg 38 in den Stützbereich 32 über. Der Stützbereich 32 ist wie ein ovaler Teller ausgebildet. Der Stützbereich 32 weist äquivalent zu dem Abstützbereich 20 eine konkave Fläche 39 auf, die an der dem Koppelbereich 33 zugewandten Seite angeordnet ist und ausgelegt ist, um den Schädelknochenabschnitt oder die Positioniervorrichtung 1 zu kontaktieren, und weist eine konvexe Fläche 40 auf, die an der dem Griffbereich 31 zugewandten Seite angeordnet ist. Der Stützbereich 32 ist durch Stege 41 gebildet, die mehrere etwa dreieckartige Aussparungen 42 voneinander trennen.

Mittig an dem Stützbereich 32 schließt der Koppelbereich 33 an. Zwischen dem Stützbereich 32 und dem Gewindebereich 34 ist eine verdünnte Stelle des Koppelbereichs 33 vorhanden, die als eine Sollbruchstelle 43 zum Schutz vor falscher Benutzung dient. Die Sollbruchstelle 43 sorgt dafür, dass der Koppelbereich 33 von dem Stützbereich 32 abbricht, wenn eine zu große Kraft in Axialrichtung oder in einer zur Axialrichtung orthogonalen oder geneigten Richtung aufgebracht wird. An die Sollbruchstelle 43 schließt der Gewindebereich 34 an, der auf das Innengewinde 30 des Gegenabschnitts 18 abgestimmt ist.

Der Gewindebereich 34 geht in einen gewindelosen Endstift 35 über. Der Endstift 35 ist so auf den Gegenabschnitt 18 abgestimmt, dass die Länge des Endstifts 35 mindestens so groß ist wie die Länge des gesamten Gegenabschnitts 18 in Längsrichtung. Dies hat zum Zweck, dass der Endstift 35 durch die Durchgangsbohrung 25 des Gegenabschnitts 18 hindurchgeführt werden kann, ohne dass das Innengewinde 30 und der Gewindebereich 34 ineinander greifen. An einem distalen Ende des Endstifts 35 ist die Verdickung 36 angeordnet. Die Durchgansbohrung 25 hat einen größeren Durchmesser als die Verdickung 36. Jedoch weist die Durchgangsbohrung 25 an einem axialen Ende (oder an einer beliebigen Stelle in der Durchgangsbohrung 25) eine Verjüngung auf, so dass die Durchgangsbohrung 25 im Bereich der Verjüngung einen kleineren Durchmesser als die Verdickung 36 des Fixierelementabschnitts 17 besitzt. Die Verdickung 36 dient somit als Verlustsicherung. Beim Einstecken des Fixierelementabschnitts 17 wird die Verdickung 36 des Fixierelementabschnitts 17 also elastisch so verformt, dass die Verdickung durch die Verjüngung/verjüngte Stelle in der Durchgangsbohrung 25 durchgeführt werden kann. Die Verdickung 36 verhindert also, dass der Fixierelementabschnitt 17 ohne Kraftaufwand, also unbeabsichtigt, durch die Durchgangsbohrung 25 des Gegenabschnitts 18 zurückrutschen kann.

Fig. 7 zeigt die beiden Abschnitte 17, 18 der Schädelknochenbefestigungsvorrichtung 16 in einem Zustand, in dem sie nicht zusammen wirken. Zum Befestigen wird der Fixierelementabschnitt 17 mit seinem Koppelbereich 33 durch die Durchgangsbohrung 25 des Gegenabschnitts 18 hindurchgesteckt. Zwischen dem Stützbereich 32 und dem Abstützbereich 20 werden die zu befestigenden Schädelknochenabschnitte mit der Positioniervorrichtung 1 zusammengeklemmt. Dabei kann der Koppelbereich 33 entweder durch die Schädelknochenabschnitte hindurchgeführt werden oder zwischen mehreren Schädelknochenabschnitten angeordnet werden. Der Koppelbereich greift also so in den Gegenabschnitt 18 ein, dass die Schädelknochenabschnitte über den Abstützbereich 20 und den Stützbereich 32 befestigt werden, da sie zwischen dem Abstützbereich 20 und dem Stützbereich 32 an der Positioniervorrichtung 1 gehalten werden.

### Bezugszeichenliste

- 1: Positioniervorrichtung
- 2: Hauptkörper
- 3: Halteelement
- 4: Rand
- 5: Tragstruktur
- 6: Steg
- 7: Aussparung/Durchgangsloch
- 8: Wabenform
- 9: Hauptbegrenzungsfläche
- 10: Nebenbegrenzungsfläche
- 11: Vorderseite
- 12: Hinterseite
- 13: Mittellinienmarkierung
- 14: Aussparung
- 15: Schräge
- 16: Schädelknochenbefestigungsvorrichtung
- 17: Fixierelementabschnitt
- 18: Gegenabschnitt
- 19: Griffbereich
- 20: Abstützbereich
- 21: Flügel
- 22: Verdickung
- 23: Vertiefung
- 24: Aussparung
- 25: Durchgangsbohrung
- 26: Konkave Fläche
- 27: Konvexe Fläche
- 28: Aussparung
- 29: Steg
- 30: Innengewinde
- 31: Griffbereich
- 32: Stützbereich
- 33: Koppelbereich
- 34: Gewindebereich
- 35: Endstift
- 36: Verdickung
- 37: Vertiefung
- 38: Steg
- 39: Konkave Fläche
- 40: Konvexe Fläche
- 41: Steg
- 42: Aussparung
- 43: Sollbruchstelle

## Patentansprüche

1. Positioniervorrichtung (1) für Schädelknochenabschnitte, zum Relativpositionieren der Schädelknochenabschnitte zueinander und zum Erhalten einer vordefinierten Gesamtform und Gesamtkontur, um eine Zielschädelform zu erreichen, mit einem zum Tragen der Schädelknochenabschnitte ausgelegten Hauptkörper (2), wobei der Hauptkörper (2), repräsentierend individuelle Patientendaten, die spezifisch für den zu behandelnden Patienten sind, und ergänzt durch modifizierte, anatomische Daten, die durch Anpassung von statistisch erfassten, unverformten Schädelformen auf verformte Stellen eines Schädels mit Berücksichtigung der Variationen der Schädelformen erzielt werden, hergestellt ist, wobei der Hauptkörper (2) eine Tragstruktur (5) aufweist,
**dadurch gekennzeichnet, dass** die Tragstruktur eine sich kreuzende Linienstruktur besitzt.

2. Positioniervorrichtung (1) nach Anspruch 1, **dadurch gekennzeichnet, dass** der Hauptkörper (2) einen umlaufenden Rand (4) besitzt, der die Tragstruktur (5) begrenzt.

3. Positioniervorrichtung (1) nach Anspruch 1 oder 2, **dadurch gekennzeichnet, dass** die Tragstruktur (5) netzartig oder gitterartig ausgeformt ist.

4. Positioniervorrichtung (1) nach einem der Ansprüche 1 bis 3, **dadurch gekennzeichnet, dass** von dem Hauptkörper (2) ein Halteelement (3) nach außen absteht, um die Positioniervorrichtung (1) zu halten, zu befestigen und/oder einzuspannen.

5. Positioniervorrichtung (1) nach Anspruch 4, **dadurch gekennzeichnet, dass** die Positioniervorrichtung (1) an einer Hinterseite eine Schräge (15) aufweist.

6. Positioniervorrichtung (1) nach einem der Ansprüche 1 bis 5, **dadurch gekennzeichnet, dass** der Hauptkörper (2) als eine konvexe Hülle ausgebildet ist.

7. Positioniervorrichtung (1) nach einem der Ansprüche 1 bis 6, **dadurch gekennzeichnet, dass** die Tragstruktur (5) eine Vielzahl von Aussparungen (7) oder Durchgangslöchern (7) besitzt, die durch Stege (6) voneinander räumlich getrennt sind.

8. Positioniervorrichtung (1) nach einem der Ansprüche 1 bis 7, **dadurch gekennzeichnet, dass** die Positioniervorrichtung (1) eine Mittellinienmarkierung (13) aufweist.

9. Herstellverfahren zum Erzeugen einer Positioniervorrichtung (1), die vorbereitet ist, um an ihr Schädelknochenabschnitte temporär zu befestigen, wobei in einem Herstellungsschritt, basierend auf Geometriedaten, die repräsentativ für die Schädelknochengeometrie des bestimmten, zu behandelnden Patienten sind, und ergänzt durch modifizierte, anatomische Daten, die durch Anpassung von statistisch erfassten, unverformten Schädelformen auf verformte Stellen eines Schädels mit Berücksichtigung der Variationen der Schädelformen erzielt werden, ein Hauptkörper (2) der Positioniervorrichtung (1) hergestellt wird, der eine Tragstruktur (5) aufweist,
**dadurch gekennzeichnet, dass** die Tragstruktur eine sich kreuzende Linienstruktur besitzt.

10. System aus der Positioniervorrichtung (1) nach einem der Ansprüche 1 bis 8 und einer Schädelknochenbefestigungsvorrichtung (16) zum temporären Befestigen eines Schädelknochenabschnitts an dem Hauptkörper (2) der Positioniervorrichtung (1), um die Schädelknochenabschnitte relativ zueinander auszurichten, **dadurch gekennzeichnet, dass** ein stiftartiger Fixierelementabschnitt (17) an der Schädelknochenbefestigungsvorrichtung (16) vorhanden ist, der zum Kontaktieren des Schädelknochenabschnitts oder mehrerer Schädelknochenabschnitte auf der einen Seite ausgelegt ist, um bei Zusammenwirken mit einem Gegenabschnitt (18) auf der anderen Seite des Schädelknochenabschnitts oder der Schädelknochenabschnitte diesen oder diese am Hauptkörper (2) der Positioniervorrichtung (1) festzulegen.

## Claims

1. Positioning device (1) for cranial bone portions for relative positioning of the cranial bone portions to each other and for obtaining a predefined overall shape and overall contour to achieve a target cranial shape, with a primary body (2) designed to support the cranial bone portions, wherein the primary body (2) is created based on representatively individual patient data specific for the patient to be treated and supplemented by modified anatomical data, which is obtained by adjusting the statistically detected, undeformed skull shapes to deformed parts of the skull, taking into account the variations of the skull shape, wherein the primary body (2) has a carrying structure (5),
**characterized in that** the carrying structure (5) has an intersecting line-structure.

2. Positioning device (1) according to claim 1, **characterized in that** the primary body (2) has a circumferential edge (4) and limits a carrying structure (5).

3. Positioning device (1) according to claim 1 or 2, **characterized in that** the carrying structure (5) has a net-like or lattice-like shape.

4. Positioning device (1) according to one of claims 1 to 3, **characterized in that** a holding element (3) protrudes outwards from the primary body (2) in order to hold, fasten, and/or clamp the positioning device (1).

5. Positioning device (1) according to claim 4, **characterized in that** the positioning device (1) has an inclination (15) on a rear side.

6. Positioning device (1) according to one of claims 1 to 5, **characterized in that** the primary body (2) is formed as a convex shell.

7. Positioning device (1) according to one of claims 1 to 6, **characterized in that** the carrying structure (5) has a plurality of recesses (7) or through-holes (7) which are spatially separated from each other by webs (6).

8. Positioning device (1) according to one of claims 1 to 7, **characterized in that** the positioning device (1) has a center line marking (13).

9. Production method for producing a positioning device (1), which is prepared to have cranial bone portions temporarily fastened to it, wherein in one manufacturing step, a primary body (2) of the positioning device (1) is produced, based on geometric data representative of the cranial bone geometry of the particular patient to be treated, and supplemented by modified anatomical data, which is obtained by adjusting the statistically detected, undeformed skull shapes to deformed parts of a skull, taking into account the variations of the skull shape, wherein the primary body (2) has a carrying structure (5),
**characterized in that** the carrying structure (5) has an intersecting line-structure.

10. System comprising a positioning device (1) according to one of claims 1 to 8 and a cranial-bone fastening device (16) for temporary fastening of a cranial bone portion to the primary body (2) of the positioning device (1) in order to orientate the cranial bone portions relative to each other, **characterized in that** a pin-shaped fixing element portion (17) is provided on the cranial-bone fastening device (16), which is designed to contact the cranial bone portion or several cranial bone portions on one side while interacting with a counter portion (18) arranged on the other side of the cranial bone portion or cranial bone portions to fasten it or them on the primary body (2) of the positioning device (1).

## Revendications

1. Dispositif de positionnement (1) pour des sections d'os crânien, destiné au positionnement relatif des sections d'os crânien les unes par rapport aux autres et à la réception d'une forme générale prédéfinie et d'un contour général prédéfini pour atteindre une forme de crâne cible, avec un corps principal (2) conçu pour porter les sections d'os crânien, dans lequel
le corps principal (2), représentant des données de patients individuelles qui sont spécifiques au patient à traiter, et augmentées par le biais de données anatomiques modifiées qui sont obtenues par le biais de l'adaptation de formes de crâne non déformées acquises statistiquement à des points déformés d'un crâne en prenant en compte les variations des formes de crâne, est produit, dans lequel le corps principal (2) présente une structure porteuse (5),
**caractérisé en ce que** la structure porteuse possède une structure à lignes qui se croisent.

2. Dispositif de positionnement (1) selon la revendication 1, **caractérisé en ce que** le corps principal (2) possède un bord périphérique (4) qui délimite la structure porteuse (5).

3. Dispositif de positionnement (1) selon la revendication 1 ou 2, **caractérisé en ce que** la structure porteuse (5) est formée à la manière d'un filet ou d'une grille.

4. Dispositif de positionnement (1) selon l'une quelconque des revendications 1 à 3, **caractérisé en ce qu'**un élément de retenue (3) dépasse du corps principal (2) vers l'extérieur pour retenir, fixer et/ou serrer le dispositif de positionnement (1).

5. Dispositif de positionnement (1) selon la revendication 4, **caractérisé en ce que** le dispositif de positionnement (1) présente une inclinaison (15) au niveau d'une face arrière.

6. Dispositif de positionnement (1) selon l'une quelconque des revendications 1 à 5, **caractérisé en ce que** le corps principal (2) est réalisé en tant qu'enveloppe convexe.

7. Dispositif de positionnement (1) selon l'une quelconque des revendications 1 à 6, **caractérisé en ce que** la structure porteuse (5) possède une pluralité d'évidements (7) ou de trous traversants (7) qui sont séparés les uns des autres dans l'espace par des entretoises (6).

8. Dispositif de positionnement (1) selon l'une quelconque des revendications 1 à 7, **caractérisé en ce que** le dispositif de positionnement (1) présente un marquage de ligne centrale (13).

9. Procédé de production destiné à la génération d'un dispositif de positionnement (1) qui est préparé pour fixer temporairement au niveau de celui-ci des sections d'os crânien, dans lequel lors d'une étape de production, sur la base de données géométriques qui sont représentatives pour la géométrie des os crâniens du patient à traiter déterminé et augmentées par le biais de données anatomiques modifiées qui sont obtenues par le biais de l'adaptation de formes de crâne non déformées acquises statistiquement à des points déformés d'un crâne en prenant en compte les variations des formes de crâne, un corps principal (2) du dispositif de positionnement (1) est produit, qui présente une structure porteuse (5),
**caractérisé en ce que** la structure porteuse possède une structure à lignes qui se croisent.

10. Système issu du dispositif de positionnement (1) selon l'une quelconque des revendications 1 à 8 et d'un dispositif de fixation d'os crânien (16) destiné à la fixation temporaire d'une section d'os crânien au niveau du corps principal (2) du dispositif de positionnement (1) pour orienter les sections d'os crânien les unes par rapport aux autres, **caractérisé en ce qu'**une section d'élément de fixation en forme de tige (17) est présente au niveau du dispositif de fixation d'os crânien (16), laquelle section de fixation est conçue pour le contact de la section d'os crânien ou de plusieurs sections d'os crânien sur l'une face pour définir lors de la coopération avec une section antagoniste (18) sur l'autre face de la section d'os crânien ou des sections d'os crânien cette ou ces sections au niveau du corps principal (2) du dispositif de positionnement (1).
